# EUROPÄISCHE PATENTANMELDUNG

(11) **EP 4 607 519 A1**
(43) Veröffentlichungstag der Anmeldung: **27.08.2025**
(21) Anmeldenummer: 24158504.1
(22) Anmeldetag: 20.02.2024
(51) Int. Cl.: G16B 30/00, H04L 67/06

(54) **COMPUTERIMPLEMENTIERTES VERFAHREN ZUR STEUERUNG EINER DATENVERARBEITUNG, VERARBEITUNGSVORRICHTUNG, COMPUTERPROGRAMM UND DATENTRÄGER**

(71) Anmelder: Siemens Healthcare Diagnostics Products GmbH, 35041 Marburg (DE)
(72) Erfinder: Hauser, Ralf, 50823 Köln (DE)
(74) Vertreter: Siemens Healthineers Patent Attorneys

(57) **Zusammenfassung**

Die Erfindung betrifft ein Verfahren zur Steuerung einer Datenverarbeitung (27) eines Eingangsdatensatzes (1, 2), wobei der jeweilige Eingangsdatensatz (1, 2) mehrere separate Dateien (7, 8) umfasst, wobei eine Übertragung des jeweiligen Eingangsdatensatzes (1, 2) zu einem Zielsystem (15) erfolgt und die Dateien (7, 8) des jeweiligen übertragenen Eingangsdatensatzes (1, 2) durch das Zielsystem (15) gespeichert werden, wobei durch das Zielsystem (15) nach dem Beginn der Übertragung eine bisherige Dateianzahl (18) der gespeicherten Dateien (7, 8) ermittelt wird, wonach in einer Schleife (19) die folgenden Schritte für den jeweiligen Eingangsdatensatz (1, 2) wiederholt werden:
- Ermitteln einer aktuellen Dateianzahl (20) der gespeicherten Dateien (7, 8),
- Prüfen einer Wiederholungsbedingung (21), die erfüllt wird oder ausschließlich erfüllbar ist, wenn die aktuelle Dateianzahl (20) die bisherige Dateianzahl (18) überschreitet, und
- Wiederholen der Schleife (19) nach einer vorgegebenen Wartezeit (22), wenn die Wiederholungsbedingung (21) erfüllt ist,
wobei bei Nichterfüllung der Wiederholungsbedingung (21),
- entweder ein den Abschluss der Übertragung betreffender Abschlusshinweis (23) an einen Benutzer (24) oder eine Freigabeeinrichtung (26) gegeben wird, oder
- entweder stets oder bei Erfüllung einer Zusatzbedingung (28) die Datenverarbeitung (27) des jeweiligen Eingangsdatensatzes (1, 2) begonnen wird.

## Beschreibung

Die Erfindung betrifft ein computerimplementiertes Verfahren zur Steuerung einer Datenverarbeitung wenigstens eines Eingangsdatensatzes, der insbesondere auf einer Analyse einer biologischen und/oder medizinischen Probe basiert, wobei der jeweilige Eingangsdatensatz mehrere separate Dateien umfasst, wobei eine Übertragung des jeweiligen Eingangsdatensatzes von einem oder einem jeweiligen Quellsystem zu einem Zielsystem erfolgt und die Dateien des jeweiligen übertragenen Eingangsdatensatzes durch das Zielsystem in einem Dateisystem des Zielsystems gespeichert werden. Daneben betrifft die Erfindung eine Verarbeitungsvorrichtung, ein Computerprogramm und einen Datenträger.

Zur Verarbeitung von medizinischen oder allgemein biologischen Daten werden mittlerweile zunehmend große Datenmengen verarbeitet und komplexe Verarbeitungsalgorithmen genutzt. Daher wird die Datenverarbeitung häufig nicht unmittelbar in einem die Daten erfassenden Labor durchgeführt, sondern separat in einem Rechenzentrum beziehungsweise durch einen Dienstleister. Beispielsweise können Rohdaten einer Sequenzierung von Genen, beispielsweise einer DNA- und/oder RNA-Sequenzierung, mehrere Gigabyte umfassen. Im Rahmen der Datenverarbeitung können in diesen Daten beispielsweise komplexe Muster erkannt beziehungsweise Zusammenhänge analysiert werden, um beispielsweise medizinischem Fachpersonal Informationen bereitzustellen, die im Rahmen einer Diagnose oder einer Therapieplanung nutzbar sind.

Mittlerweile erfolgt typischerweise eine NGS-Sequenzierung (Next Generation Sequencing, NGS), bei der beispielsweise simultan mehrere hundert Millionen Fragmente in einer Probe sequenziert werden und somit hohe Sequenzierungsdurchsätze erreicht werden können. Die Sequenzierungsrohdaten werden in einer spezifischen Datenstruktur gespeichert. Unterschiedliche Sequenzierungsläufe können sich in der Anzahl und Größe der erzeugten Dateien unterscheiden, beispielsweise in Abhängigkeit davon, welches Panel im Laborprozess verwendet wird. Zusätzlich ist die Anzahl der Proben, die in einem einzelnen Sequenzierungslauf verarbeitet werden, ein Faktor für die Größe der Datenpakete.

Um eine Datenverarbeitung durch ein externes Rechenzentrum beziehungsweise einen Dienstleister zu ermöglichen, werden die Rohdaten an ein sicheres, externes Dateisystem übertragen. Dort kann die die bioinformatische Pipeline implementierende Datenverarbeitungseinrichtung die Genomdaten verarbeiten beziehungsweise eine solche Datenverarbeitungseinrichtung kann die Datenverarbeitung aus dem externen Dateisystem auslesen.

Je nach Größe und Konnektivität dauert die Übertragung eines solchen Rohdatensatzes an das externe Dateisystem mehrere Stunden. Um die Zeit, die von der Sequenzierung bis zur Verarbeitung der Sequenzierungsdaten benötigt wird, zu minimieren, sollte die bioinformatische Pipeline so schnell wie möglich gestartet werden. Daher kann mit der Übertragung der Daten bereits während der Laufzeit des Sequenzierautomats begonnen werden.

Bei einem Sequenzierautomat beziehungsweise allgemein bei Datenverarbeitungssystemen in Laboren, die, insbesondere personenbezogene, medizinische beziehungsweise biologische Daten zur Verfügung stellen, handelt es sich jedoch um Datenverarbeitungssysteme, die typischerweise besonders hohen Anforderungen bezüglich der Datensicherheit und der Datenvertraulichkeit genügen müssen, da dort potenziell hochsensible personenbezogene Informationen gehandhabt werden. Daher ist es in vielen Anwendungsfällen ausgeschlossen, dass die Datenverarbeitungseinrichtung, die die bioinformatische Pipeline implementiert und die beispielsweise auch als Cloudlösung implementiert sein kann, unmittelbar mit dem Sequenzierautomat kommuniziert, um einen Abschluss der Sequenzierung und somit einen geeigneten Zeitpunkt zum Beginn der Datenverarbeitung zu erkennen.

Aufgrund dieses fehlenden Kommunikationskanals und des obig erläuterten variablen Umfangs der übertragenen Daten verfügt die Datenverarbeitungseinrichtung typischerweise auch über keinerlei Informationen über die Anzahl und Größe der Dateien, die für den jeweiligen Sequenzierungslauf erwartet werden.

Dies führt dazu, dass bislang die übertragenen Sequenzierungsrohdaten manuell auf Vollständigkeit geprüft werden müssen, bevor die bioinformatische Pipeline gestartet wird. Hierzu benötigt eine Vertrauensperson, beispielsweise ein IT-Techniker, sowohl Zugriffsrechte auf und eine Verbindung zu dem externen Dateisystem als auch Zugriff auf das Quellsystem, also den Sequenzierautomat beziehungsweise das Datenverarbeitungssystem des Labors. Die Vertrauensperson wird seitens eines Laborbeschäftigten über den Beginn der Sequenzierung beziehungsweise der Datenübertragung informiert. Nach einer angemessenen Wartezeit prüft die Vertrauensperson manuell und potenziell auch mehrfach, ob die Anzahl der Dateien und deren Größe auf dem externen Dateisystem mit diesen Größen im Quellsystem übereinstimmt. Sobald dies der Fall ist, startet die Vertrauensperson die bioinformatische Pipeline manuell beziehungsweise informiert eine weitere Person, dass die Verarbeitung gestartet werden kann. Es handelt sich somit um einen vollständig manuellen Prozess.

Aus dem geschilderten Vorgehen resultieren mehrere verschiedene technische Nachteile beziehungsweise Probleme. Zum einen führt dieses Vorgehen zu relativ langen Antwortzeiten des Gesamtsystems, also Zeitspannen vom Beginn der Sequenzierung bis zum Vorliegen des Verarbeitungsergebnisses, da die manuelle Überprüfung, ob die Dateiübertragung abgeschlossen ist, häufig erst deutlich nach dem tatsächlichen Abschluss der Dateiübertragung erfolgt und anschließend noch weitere Verzögerungen bis zum Starten der externen Datenverarbeitung resultieren können, insbesondere wenn nicht alle beteiligten Stellen durchgehend besetzt sind und beispielsweise die Datenübertragung erst nachts abgeschlossen ist. Zum anderen kann dieses Vorgehen zu einer ineffizienten Nutzung der externen Datenverarbeitungseinrichtung führen, da freie Ressourcen potenziell nicht genutzt werden, obwohl tatsächlich bereits die Verarbeitung von Rohdaten einer weiteren Sequenzierung möglich wäre, da für diese die Datenübertragung bereits abgeschlossen ist.

Der Erfindung liegt somit die Aufgabe zugrunde, die Antwortzeit und die Effizienz einer bioinformatischen Datenverarbeitung, insbesondere der Verarbeitung von Gensequenzdaten, weiter zu verbessern.

Die Aufgabe wird erfindungsgemäß durch ein computerimplementiertes Verfahren eingangs genannter Art gelöst, wobei durch das Zielsystem nach dem Beginn der Übertragung eine bisherige Dateianzahl der in dem Dateisystem gespeicherten Dateien des jeweiligen Eingangsdatensatzes ermittelt wird, wonach in einer Schleife die folgenden Schritte für den jeweiligen Eingangsdatensatz wiederholt werden:
- Ermitteln einer aktuellen Dateianzahl der in dem Dateisystem gespeicherten Dateien des jeweiligen Eingangsdatensatzes,
- Prüfen einer Wiederholungsbedingung, die erfüllt wird oder ausschließlich erfüllbar ist, wenn die aktuelle Dateianzahl die bisherige Dateianzahl überschreitet, und
- Wiederholen der Schleife nach einer vorgegebenen Wartezeit, wenn die Wiederholungsbedingung erfüllt ist, wobei in dem nachfolgenden Schleifendurchlauf als bisherige Dateianzahl die in dem aktuellen Schleifendurchlauf oder die in einem früheren Schleifendurchlauf erfasste aktuelle Dateianzahl verwendet wird,
wobei bei Nichterfüllung der Wiederholungsbedingung,
- entweder ein den Abschluss der Übertragung betreffender Abschlusshinweis an einen Benutzer oder eine Freigabeeinrichtung gegeben wird, wobei nach einer Freigabe durch den Benutzer oder die Freigabeeinrichtung die Datenverarbeitung des jeweiligen Eingangsdatensatzes begonnen wird, oder
- entweder stets oder bei Erfüllung einer Zusatzbedingung die Datenverarbeitung des jeweiligen Eingangsdatensatzes begonnen wird.

Im Rahmen der Datenverarbeitung können dann zumindest Teile des jeweiligen Eingangsdatensatzes aus dem Dateisystem ausgelesen werden.

Wie bereits eingangs erläutert wurde, soll aus Gründen der Datensicherheit und -vertraulichkeit häufig kein externer Zugriff auf bestimmte Quellsysteme, insbesondere auf Sequenzierautomaten und andere Vorrichtungen der Labormesstechnik, möglich sein. Soweit eine laborexterne Datenverarbeitung erfolgen soll, ist das Quellsystem daher vorzugsweise derart mit der Außenwelt vernetzt, dass ausschließlich ein Datentransfer vom Quellsystem zur Außenwelt hin, jedoch kein Datentransfer von der Außenwelt zum Quellsystem erfolgen kann. Dies kann beispielsweise durch eine geeignete Konfiguration einer Firewall erreicht werden.

Zur Steuerung der Datenverarbeitung stehen somit typischerweise ausschließlich der jeweilige Eingangsdatensatz selbst beziehungsweise die von diesem umfassten Dateien zur Verfügung. Da für Sequenzierautomaten und andere Labortechnik die Art der Datenbereitstellung jedoch häufig nur eingeschränkt konfigurierbar ist, also beispielsweise nur die Auswahl eines Zieldateisystems möglich ist, kann der Abschluss einer Sequenzierung oder einer anderen Messaufgabe häufig nicht an eine externe Einrichtung signalisiert werden.

Im Rahmen der Erfindung wurde jedoch erkannt, dass, insbesondere für Quellsysteme aus dem Bereich der Sequenzierung beziehungsweise allgemein der Labortechnik, davon ausgegangen werden kann, dass der zeitliche Abstand der Übertragung von nacheinander übertragenen Dateien und somit auch der Abstand von deren Speicherung im zielsystemseitigen Dateisystem unterhalb einer bekannten Zeitobergrenze liegt. Im Rahmen der Entwicklung der Erfindung wurde beispielhaft das Übertragungsverhalten verschiedener Sequenzierautomaten analysiert und erkannt, dass bei einer Übertragung von Dateien zu einem externen Dateisystem typischerweise alle 10 Sekunden bis 4 Minuten eine neue Datei erzeugt wird.

Wird somit im erfindungsgemäßen Verfahren eine hinreichend große Wartezeit, beispielsweise eine Wartezeit von 5 Minuten bis 10 Minuten, genutzt, so kann ein Abschluss der Übertragung des jeweiligen Eingangsdatensatzes robust erkannt werden und somit der Beginn der Datenverarbeitung, insbesondere automatisch, ausgelöst beziehungsweise hierfür zunächst eine Freigabe von dem Benutzer beziehungsweise der Freigabeeinrichtung eingeholt werden.

Statt einer Freigabe durch einen Benutzer beziehungsweise eine Freigabeeinrichtung kann auch durch das Zielsystem selbst beziehungsweise allgemein ein System, das die Datenverarbeitung des auf dem Zielsystem gespeicherten Eingangsdatensatzes durchführt, die Zusatzbedingung ausgewertet werden, die anhand von Parametern des Eingangsdatensatzes beziehungsweise der Dateien des Eingangsdatensatzes prüfen beziehungsweise abschätzen kann, ob die Übertragung erfolgreich war.

Prinzipiell kann die Schleife auch mit einer Wartezeit wiederholt werden, die kürzer ist als der maximale erwartete Zeitabstand zwischen Dateitransfers vom Quellsystem. In diesem Fall kann als bisherige Dateianzahl beispielsweise die Dateianzahl des vorletzten oder eines noch früheren Schleifendurchlaufs herangezogen werden. Hierdurch kann auch in diesem Fall sichergestellt werden, dass zwischen der Erfassung der bisherigen und der aktuellen Dateianzahl ein Zeitintervall liegt, das hinreichend lang ist, sodass bei noch nicht abgeschlossener Übertragung des jeweiligen Eingangsdatensatzes davon ausgegangen werden kann, dass in diesem Zeitintervall eine weitere Datei in dem Dateisystem abgelegt wird.

Obwohl das der Erfindung zugrunde liegende Problem eingangs am konkreten Beispiel der Übertragung und Auswertung von Rohdaten einer Gensequenzierung beschrieben wurde, ist das erfindungsgemäße Verfahren auch auf beliebige andere Prozesse übertragbar, in denen medizinische beziehungsweise biologische Daten aus einem Bereich besonders kritischer Datensicherheit beziehungsweise Datenvertraulichkeit zu einem separaten Datenspeicher übertragen werden sollen, um dort verarbeitet beziehungsweise zur Verarbeitung ausgelesen zu werden.

Bei dem Eingangsdatensatz kann es sich neben den bereits erwähnten Rohdaten einer Sequenzierung beispielsweise auch um beliebige andere Labordaten, zum Beispiel um Blutwerte oder um medizinische Bilddaten, handeln. Bevorzugt umfasst der jeweilige übertragene Eingangsdatensatz Messwerte oder aus Messwerten ermittelte Größen.

Die Datenverarbeitung des jeweiligen Eingangsdatensatzes kann unmittelbar durch das Zielsystem erfolgen. Es ist jedoch auch möglich, dass ein weiteres System, das auf das Dateisystem des Zielsystems lesend und vorzugsweise auch schreibend zugreifen kann, die Datenverarbeitung durchführt.

Das Quellsystem kann insbesondere ein Sequenzierautomat sein. Es ist jedoch beispielsweise auch möglich, dass das Quellsystem mehrere Datenquellen, beispielsweise mehrere Sequenzierautomaten und/oder weitere Einrichtungen der Labormesstechnik, umfasst. Daten der Datenquellen können in diesem Fall beispielsweise durch einen Server des Quellsystems, beispielsweise einen zentralen Server eines Labors, zusammengeführt werden. Es ist auch möglich, dass als Quellsystem ein dezentraler Rechnerverbund genutzt wird, beispielsweise ein virtuelles privates Netz (VPN, auch englisch "virtual private network") eines Laborverbundes.

Das Zielsystem kann beispielsweise als Cloudlösung implementiert sein oder durch einen Server oder einen Server-Cluster oder einen Arbeitsplatzrechner.

Die Gabe des Abschlusshinweises an den Benutzer beziehungsweise die Freigabeeinrichtung kann insbesondere dazu dienen, eine Freigabe anzufordern. Hierbei ist es möglich, dass der Abschlusshinweis wenigstens eine Information, beispielsweise die bei Abbruch der Schleife zuletzt erfasste aktuelle Dateianzahl und/oder das Datenvolumen des empfangenen Eingangsdatensatzes und/oder der einzelnen empfangenen Dateien, umfasst. Diese Information kann beispielsweise an den Benutzer ausgegeben werden, sodass dieser auf Basis der Information entscheiden kann, ob die Freigabe erfolgen soll oder ob voraussichtlich ein Übertragungsfehler vorliegt.

Bei einer Übertragung des Abschlusshinweises an die Freigabeeinrichtung kann eine automatische Auswertung der Information erfolgen, wobei beispielsweise geprüft werden kann, ob die Dateianzahl und/oder das jeweilige Datenvolumen innerhalb eines erwarteten jeweiligen Intervalls liegt.

Die Gabe des Abschlusshinweises kann jedoch auch dazu dienen, den Benutzer beziehungsweise die Freigabeeinrichtung über einen fehlerhaften Abschluss der Übertragung zu informieren. Beispielsweise kann das Zielsystem, beispielsweise im Rahmen der Datenübertragung und/oder der Speicherung der Dateien im Dateisystem, eine Fehlererkennung, beispielsweise auf Basis einer Prüfsumme, einer Signaturprüfung und/oder eines CRC-Codes, durchführen. Schlägt diese fehl, so kann die Wiederholungsbedingung nicht erfüllt seien und eine diesbezügliche Information kann als Abschlusshinweis an den Benutzer beziehungsweise die Freigabeeinrichtung ausgegeben werden.

Die Hinweisgabe kann beispielsweise dadurch erfolgen, dass eine E-Mail, eine SMS oder allgemein eine Nachricht gemäß eines beliebigen Nachrichtenübertragungsprotokolls an den Benutzer beziehungsweise ein dem Benutzer zugeordnetes Endgerät versandt wird. Zur Gabe des Abschlusshinweises können jedoch auch beliebige Protokolle zur Kommunikation zwischen vernetzten Geräten beziehungsweise Diensten genutzt werden.

Werden mehrere Eingangsdatensätze übertragen, so kann die Übertragung der einzelnen Eingangsdatensätze sequenziell nacheinander erfolgen. Es ist jedoch auch möglich, dass die Zeiträume, während denen verschiedene der Eingangsdatensätze übertragen werden, zumindest teilweise überlappen. Dies kann insbesondere der Fall sein, wenn die Eingangsdatensätze von unterschiedlichen Quellsystemen übertragen werden.

Eine Zuordnung der übertragenen Daten beziehungsweise Dateien zur einem der übertragenen Eingangsdatensätze ist in diesem Fall weiterhin möglich, beispielsweise wenn die übertragenen Datenpakete beziehungsweise Dateien eine eindeutige Identifikation aufweisen, die eine Zuordnung zu einem der Eingangsdatensätze ermöglicht und/oder wenn verschiedene der Eingangsdatensätze von unterschiedlichen Quellsystemen übertragen werden, wodurch Daten, die von einem bestimmten Quellsystem übertragen werden, eindeutig einem der Eingangsdatensätze zugeordnet werden können. Beispielsweise wird bei Sequenzierautomaten einzelnen Sequenzierläufen typischerweise eine sogenannte "RunID" zugeordnet, die als eindeutige Identifikation verwendet werden kann.

Erfolgt eine Steuerung der Datenverarbeitung mehrerer Eingangsdatensätze, so kann seitens des Zielsystems jeweils eine Initialisierungsfunktion ausgeführt werden, wenn erkannt wird, dass erstmalig eine Datei oder Teildaten einer Datei eines der Eingangsdatensätze empfangen werden.

Die Erkennung, dass erstmalig eine Datei oder Teildaten einer Datei der Eingangsdaten empfangen werden, ist dann besonders einfach möglich, wenn die übertragenen Datenpakete beziehungsweise Dateien, wie obig erläutert, eine eindeutige Identifikation aufweisen. In diesem Fall kann beispielsweise in einer Log-Datei oder, wie später noch erläutert wird, durch Anlegen eines separaten Ordners für die Dateien des jeweiligen Eingangsdatensatzes, vermerkt werden, dass zu einem Eingangsdatensatz bereits wenigstens eine Datei beziehungsweise Teildaten empfangen wurden.

Eine Erkennung, dass erstmalig eine Datei oder Teildaten einer Datei der Eingangsdaten empfangen werden, ist jedoch auch ohne eine solche eindeutige Identifikation möglich, beispielsweise wenn davon ausgegangen werden kann, dass von einem einzigen Quellsystem, beispielsweise einem einzigen Sequenzierautomaten, keine parallele Übertragung unterschiedlicher Eingangsdatensätze erfolgt. Da im erfindungsgemäßen Verfahren der Abschluss der Übertragung eines jeweiligen Eingangsdatensatzes erkannt wird, kann bei einem erneuten Datenempfang von einem Quellsystemen, dessen Übertragung vorangehend bereits abgeschlossen war, davon ausgegangen werden, dass erstmalig Teildaten eines neuen Eingangsdatensatzes übertragen werden.

Im Rahmen der Initialisierungsfunktion kann ein dem jeweiligen Eingangsdatensatz zugeordneter Ordner in dem Dateisystem angelegt werden, wobei die Dateien des jeweiligen Eingangsdatensatzes in diesem Ordner abgelegt werden. Durch Verwendung separater Ordner für Dateien unterschiedlicher Eingangsdatensätze wird eine effiziente Datenverarbeitung erreicht, insbesondere da dies eine einfache und robuste Verwaltung von Zugriffsrechten ermöglicht. Zudem ist durch Nutzung der separaten Ordner die Dateianzahl besonders einfach ermittelbar.

Im Rahmen der Initialisierungsfunktion kann ergänzend oder alternativ ein Initialisierungshinweis an den Benutzer und/oder einen weiteren Benutzer und/oder die Freigabeeinrichtung und/oder eine andere Einrichtung gegeben werden. Der Initialisierungshinweis kann auf verschiedene Weisen gegeben werden, wie bereits obig zum Abschlusshinweis erläutert wurde. Die Übertragung des Initialisierungshinweises kann als Bestätigung dienen, dass die Übertragung des Eingangsdatensatzes begonnen hat. Dies ist insbesondere zweckmäßig, wenn die Übertragung des Eingangsdatensatzes bereits während der Datenerfassung, beispielsweise während der Sequenzierung, beginnt, da in diesem Fall der Initialisierungshinweis beispielsweise den Auftraggeber einer Gensequenzierung beziehungsweise ein System des Auftraggebers informieren kann, dass die beauftragte Gensequenzierung begonnen hat, ohne dass eine separate Kommunikation mit dem Quellsystem erforderlich ist.

Ergänzend oder alternativ kann im Rahmen der Initialisierungsfunktion ein aktueller Zeitpunkt als Referenzzeitpunkt festgelegt werden, wobei ein Fehlerhinweis an den Benutzer und/oder den oder einen weiteren Benutzer und/oder die Freigabeeinrichtung und/oder die oder eine andere Einrichtung gegeben wird, wenn ein Zeitabstand zu dem Referenzzeitpunkt überschritten wird, bevor der Abschlusshinweis gegeben und/oder die Datenverarbeitung des jeweiligen Eingangsdatensatzes begonnen wird.

Beispielsweise kann der Referenzzeitpunkt festgelegt werden, indem ein dem jeweiligen Eingangsdatensatz zugeordneter Zeitzähler auf einen ersten Wert gesetzt wird, wobei der Zeitzähler beispielsweise angehalten oder zurückgesetzt werden kann, wenn der Abschlusshinweis gegeben beziehungsweise die Datenverarbeitung begonnen wird. Der Fehlerhinweis kann dann gegeben werden, sobald dieser Zeitzähler einen zweiten Wert erreicht beziehungsweise über- oder unterschreitet. Insbesondere bei einem zeitlich überlappenden Empfang von mehreren Eingangsdatensätzen kann es jedoch einfacher sein, beispielsweise eine Uhr des Zielsystems zu nutzen, um den Referenzzeitpunkt festzulegen und dann beispielsweise in regelmäßigen Abständen zu prüfen, ob der Zeitabstand zu dem gespeicherten Referenzzeitpunkt überschritten wird.

Beispielsweise kann ein Fehlerhinweis gegeben werden, wenn die Datenübertragung nach einem Tag oder einem anderen vorgegebenen Zeitraum noch nicht abgeschlossen ist. Die Gabe des Fehlerhinweises kann beispielsweise durch ein Interruptsignal des Zeitzählers ausgelöst werden, oder die Zeit kann durch einen parallelen und von der Schleife unabhängigen Prozess überwacht werden, sodass dies quasi als Watchdog-Timer dient, der die korrekte Funktion des die Schleife implementierenden Programmteils überwacht. Somit kann robust erkannt werden, wenn die Übertragung des Eingangsdatensatzes beziehungsweise die Überwachung der Dateianzahl fehlschlägt.

Der Abschlusshinweis kann die zuletzt erfasste aktuelle Dateianzahl und/oder ein Datenvolumen des jeweiligen Eingangsdatensatzes umfassen. Die genannten Informationen ermöglichen es dem Benutzer beziehungsweise der Freigabeeinrichtung abzuschätzen, ob die Dateianzahl beziehungsweise das Datenvolumen innerhalb eines jeweiligen Erwartungsintervalls liegen. Zwar können die Dateianzahl beziehungsweise das Datenvolumen zwischen verschiedenen Übertragungen stark schwanken, wie bereits eingangs erläutert wurde. Diese Größen liegen jedoch typischerweise in einem gewissen Rahmen. Beispielsweise kann davon ausgegangen werden, dass die im Rahmen eines Sequenzierungslaufs anfallenden Eingangsdaten ein Datenvolumen zwischen 4 GB und 50 GB aufweisen, sodass beispielsweise bei einem Datenvolumen von weniger als 3 GB oder von mehr als 150 GB von einer fehlerhaften Übertragung ausgegangen werden kann und die Freigabe unterbleiben kann beziehungsweise zunächst eine manuelle Prüfung der übertragenen Daten erfolgen kann.

Die Freigabeeinrichtung kann eine Freigabebedingung auswerten, deren Erfüllung davon abhängt, ob die zuletzt erfasste aktuelle Dateianzahl und/oder das Datenvolumen innerhalb eines jeweiligen vorgegebenen Erwartungsintervalls liegen. Die Freigabebedingung kann insbesondere erfüllt werden oder nur dann erfüllbar sein, wenn die Dateianzahl und/oder das Datenvolumen innerhalb des jeweiligen vorgegebenen Erwartungsintervalls liegen. Bei Erfüllung der Freigabebedingung kann die Freigabeeinrichtung die Datenverarbeitung des jeweiligen Eingangsdatensatzes freigeben.

Die Datenverarbeitung kann somit insbesondere nur dann beginnen, wenn für die vorgegebenen Wartezeit keine neuen Dateien des jeweiligen Eingangsdatensatzes empfangen werden, was auf einen Abschluss der Übertragung des jeweiligen Eingangsdatensatzes hinweist, und zudem die Anzahl der insgesamt zu dem Eingangsdatensatz empfangenen Dateien beziehungsweise deren Datenvolume im Erwartungsintervall liegt, womit auch die Dateizahl bzw. -größe eine vollständige Übertragung indizieren.

Beginnt die Datenverarbeitung bei Erfüllung der Zusatzbedingung, so kann die Erfüllung der Zusatzbedingung davon abhängen, ob die zuletzt erfasste aktuelle Dateianzahl und/oder ein Datenvolumen des jeweiligen Eingangsdatensatzes innerhalb eines jeweiligen vorgegebenen Erwartungsintervalls liegen. Abgesehen davon, dass die Prüfung der Zusatzbedingung beispielswese auch lokal seitens des Zielsystems erfolgen kann und somit keine Übertragung der entsprechenden Informationen beispielsweise an eine Freigabeeinrichtung erforderlich ist, kann die Auswertung der Zusatzbedingung somit der bereits obig erläuterten Auswertung der Freigabebedingung entsprechen, sodass auch in diesem Falle die genannten Vorteile erreicht werden können.

Der jeweilige Eingangsdatensatz kann durch eine Analyse einer biologischen und/oder medizinischen Probe durch das Quellsystem ermittelt werden, wobei die Übertragung des jeweiligen Eingangsdatensatzes beginnt, bevor die Analyse der biologischen und/oder medizinischen Probe abgeschlossen ist. Wie bereits obig erläutert wurde, kann die Übertragung des jeweiligen Eingangsdatensatzes sehr zeitaufwändig sein. Um die Zeit zwischen dem Beginn der Analyse der Probe und dem Erhalten des Ergebnisses der Datenverarbeitung zu minimieren, kann die Datenübertragung daher zumindest zum Teil parallel zur Durchführung der Analyse durch das Quellsystem erfolgen.

Die Eingangsdaten können durch Sequenzierung wenigstens eines Teils wenigstens eines Genoms in einer biologischen und/oder medizinischen Probe bereitgestellt werden. Die Sequenzierung eines Genoms stellt einen Sonderfall der Analyse einer biologischen und/oder medizinischen Probe dar. Da bei einer solchen Analyse verglichen mit anderen Laboruntersuchungen besonders große Datenmengen auftreten können, womit die Datenübertragung relativ viel Zeit in Anspruch nimmt, ist das erfindungsgemäße Vorgehen in diesem Fall besonders relevant.

Neben dem erfindungsgemäßen computerimplementierten Verfahren betrifft die Erfindung eine Datenverarbeitungseinrichtung, die zur Teilnahme an dem erfindungsgemäßen computerimplementierten Verfahren als das Zielsystem eingerichtet ist.

Das Zielsystem und somit die erfindungsgemäße Datenverarbeitungseinrichtung implementiert vorzugsweise zumindest ein Empfangen des Eingangsdatensatzes beziehungsweise von dessen Dateien im Rahmen der Übertragung des jeweiligen Eingangsdatensatzes, die innerhalb der Schleife durchgeführten Schritte sowie einerseits die Gabe des Abschlusshinweises und/oder andererseits die Durchführung der Datenverarbeitung und optional die Prüfung der Zusatzbedingung.

Rein beispielhaft kann das Zielsystem über den Cloud-Dienst Azure^{®} Data Factory implementiert werden, wobei die Überprüfung auf den Abschluss der Übertragung in der Schleife und die Hinweisgabe beziehungsweise das Auslösen der Datenverarbeitung durch ein geeignetes Benutzerprogramm in den Cloud-Dienst integriert sein können.

Auch die mit Bezug auf Weiterbildungen des erfindungsgemäßen Verfahrens erläuterten Schritte können durch die Datenverarbeitungseinrichtung beziehungsweise das Zielsystem implementiert werden. Generell sind zum erfindungsgemäßen Verfahren offenbarte Merkmale mit den dort genannten Vorteilen auf die erfindungsgemäße Datenverarbeitungseinrichtung übertragbar und umgekehrt.

Im einfachsten Fall kann die Datenverarbeitungseinrichtung durch einen Server implementiert sein. Besonders bevorzugt ist die Verarbeitungsvorrichtung jedoch als Cloudlösung oder auch als Gruppe mehrerer Server implementiert. Soweit die Datenverarbeitung des jeweiligen Eingangsdatensatzes nicht durch das Zielsystem beziehungsweise die dieses implementierende Datenverarbeitungseinrichtung selbst erfolgt, kann die Datenverarbeitung beispielsweise durch die Freigabeeinrichtung nach Empfang des Abschlusshinweises erfolgen. In diesem Fall sollte jedoch vorzugsweise ein Zugriff der Freigabeeinrichtung auf das Zielsystem mit hoher Datenrate möglich sein, um eine zügige Datenverarbeitung zu ermöglichen.

Zudem betrifft die Erfindung ein Computerprogramm mit Anweisungen, die dazu eingerichtet sind, bei einer Ausführung auf einer Datenverarbeitungseinrichtung die durch das Zielsystem implementierten Schritte des erfindungsgemäßen computerimplementierten Verfahrens durchzuführen.

Zudem betrifft die Erfindung einen Datenträger, der ein erfindungsgemäßes Computerprogramm umfasst.

Die zum erfindungsgemäßen Verfahren beziehungsweise die zur erfindungsgemäße Datenverarbeitungseinrichtung offenbarten Merkmale lassen sich mit den dort genannten Vorteilen auch auf das erfindungsgemäße Computerprogramm und/oder den erfindungsgemäßen Datenträger übertragen und umgekehrt.

Weitere Vorteile und Einzelheiten der Erfindung ergeben sich aus den folgenden Ausführungsbeispielen sowie den zugehörigen Zeichnungen. Hierbei zeigen schematisch:
- Fig. 1: ein Ablaufdiagramm eines Ausführungsbeispiels des erfindungsgemäßen computerimplementierten Verfahrens, und
- Fig. 2: einen Ausführungsbeispiel einer erfindungsgemäßen Datenverarbeitungseinrichtung.

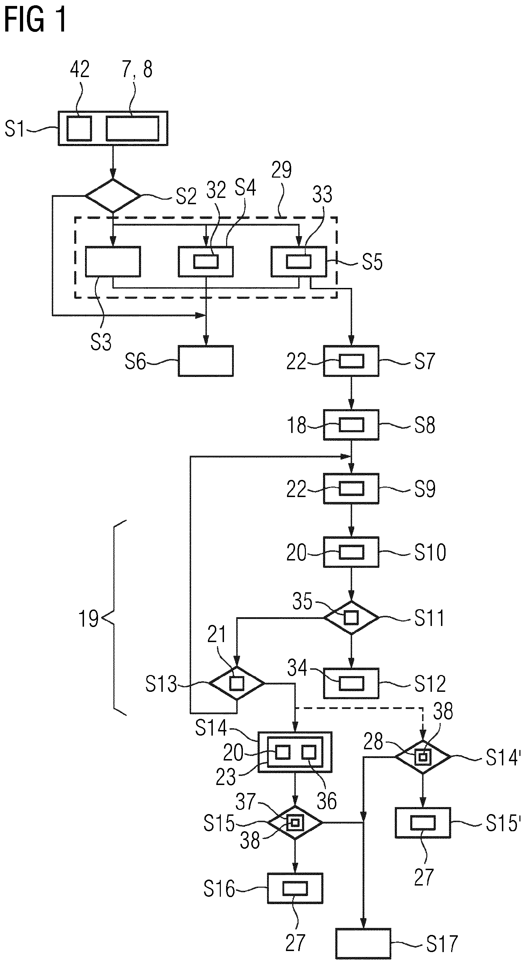

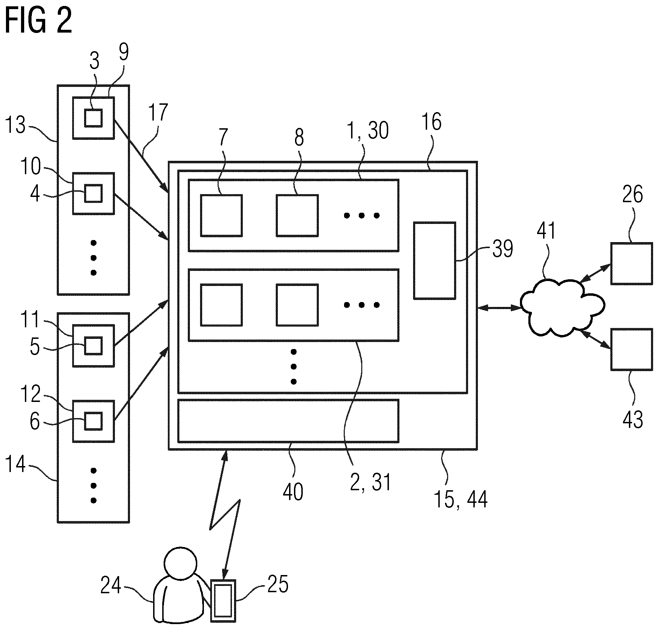

Fig. 1 zeigt ein Ausführungsbeispiel eines Verfahrens zur Steuerung der Datenverarbeitung 27 von Eingangsdatensätzen 1, 2. Zum besseren Verständnis wird das Verfahren im Folgenden mit zusätzlichem Bezug auf Fig. 2 erläutert, die im Beispiel zur Verfahrensdurchführung genutzte Komponenten und Datenstrukturen zeigt. Zum besseren Verständnis soll hierbei zunächst ein Überblick über das gezeigte Verfahren gegeben werden, bevor die einzelnen Schritte diskutiert werden.

In dem Verfahren soll insbesondere erkannt werden, ob ein jeweiliger zu verarbeitender Eingangsdatensatz 1, 2 von einem Zielsystem 15 bereits vollständig empfangen wurde. Das Zielsystem 15 empfängt im Beispiel Eingangsdatensätze 1, 2 von einer Vielzahl von Quellsystemen 9-12, die im Beispiel Sequenzierautomaten sind, die in verschiedenen Laboren 13, 14 angeordnet sind. Im Beispiel wird der jeweilige Eingangsdatensatz 1, 2 somit durch eine Analyse einer biologischen und/oder medizinischen Probe 3-6 durch das jeweilige Quellsystem 9-12 ermittelt. Konkret werden die Eingangsdaten 1, 2 hierbei im Beispiel durch eine Sequenzierung wenigstens eines Teils wenigstens eines Genoms der biologischen und/oder medizinischen Probe 3-6 bereitgestellt.

Um ein Verarbeitungsergebnis möglichst zeitnah nach dem Beginn der Analyse der jeweiligen Probe 3-6 zu erhalten, soll die Verarbeitung des jeweiligen Eingangsdatensatzes 1, 2 in dem Zielsystem 15 möglichst frühzeitig beginnen. Um dies zu ermöglichen, beginnt im Beispiel einerseits die Übertragung des jeweiligen Eingangsdatensatzes 1, 2 bereits, bevor die Analyse der jeweiligen biologischen und/oder medizinischen Probe 3-6 abgeschlossen ist. Zudem soll seitens des Zielsystems 15 automatisiert erkannt werden, wenn der jeweilige Eingangsdatensatz 1,2 vollständig übertragen ist, um möglichst verzögerungsfrei mit der Verarbeitung beginnen zu können.

In dem in Fig. 1 gezeigten Ausführungsbeispiel erfolgt eine Überwachung, ob die Übertragung des jeweiligen Eingangsdatensatz 1, 2 abgeschlossen ist, in den Schritten S7 bis S13. Hierbei wird in einer Schleife 19 nach einer vorgegebenen Wartezeit 22 jeweils geprüft, ob eine aktuelle Dateianzahl 20 der für den jeweiligen Eingangsdatensatz 1, 2 in dem Dateisystem 16 des Zielsystems 15 gespeicherten Dateien 7,8 die bisherige Dateianzahl 18 dieser Dateien überschreitet. Ist dies für eine hinreichend lange Wartezeit 22 nicht der Fall, so wird davon ausgegangen, dass die Übertragung des jeweiligen Eingangsdatensatzes 1, 2 vollständig abgeschlossen ist. In diesem Fall wird in den Schritten S 14 bis S16 ein Abschlusshinweis 23 ausgegeben, und eine Freigabe für die Datenverarbeitung 27 eingeholt beziehungsweise in der alternativen Ausgestaltung gemäß der Schritte S14' und S15' wird in diesem Fall bei Erfüllung einer Freigabebedingung 37 automatisch mit der Datenverarbeitung 27 begonnen.

Die Schritte S7 bis S13 werden für alle Eingangsdatensätze 1, 2, deren Übertragung aktuell noch nicht abgeschlossen ist, parallel zueinander und zudem parallel zum Empfang und Speichern der Dateien 7, 8 der verschiedenen Eingangsdatensätze 1, 2, also zu den Schritten S1 bis S6, durchgeführt. Die Schritte S14 bis S16 werden dann durchgeführt, wenn der jeweilige Eingangsdatensatz 1, 2 anscheinend vollständig übertragen ist. Fig. 1 zeigt zudem die Schritte S14' und S15', die alternativ zu den Schritten S 14 bis 16 nutzbar sind.

Im Folgenden werden die einzelnen Verfahrensschritte des Beispiels im Detail erläutert. Schritt S1 wird hierbei jedes Mal ausgeführt, wenn durch das Zielsystem 15 eine Datei 7, 8 von einem der Quellsysteme 9-12 empfangen wird. Wie durch die Pfeile 17 schematisch dargestellt ist, ist die Datenverbindung zwischen den Quellsystemen 9-12 und dem Zielsystem unidirektional, sodass durch das Zielsystem 15 beispielsweise keine Rückfragen bei den Quellsystemen 9-12 möglich sind, ob deren Probenanalyse bereits abgeschlossen ist.

Die Übertragung der jeweiligen Datei 7, 8 beziehungsweise von Teildaten kann durch übliche Übertragungsprotokolle, beispielsweise über gängige Internet- beziehungsweise allgemein Netzwerkprotokolle, Peer-to-Peer-Protokolle oder beliebige andere Übertragungsprotokolle erfolgen. Vorzugsweise ist die Datenübertragung vor Dritten geschützt, beispielsweise durch Nutzung eines Virtual Private Network. Als Teil der jeweiligen Übertragung wird im Beispiel auch eine Kennung 42 des jeweiligen Eingangsdatensatzes, also beispielsweise eine "RunID", übertragen. Wie bereits im allgemeinen Teil diskutiert wurde, wäre es jedoch auch ausreichend, ausschließlich eine Kennung des Quellsystems zu übertragen, wenn davon ausgegangen werden kann, dass ein jeweiliges Quellsystem 9-12 zeitgleich nur einen einzigen Eingangsdatensatz 1, 2 überträgt.

In Schritt S2 wird dann überprüft, ob erstmalig eine Datei 7, 8 beziehungsweise Teildaten einer Datei 7, 8 für einen Eingangsdatensatz 1, 2 empfangen werden. Da im Beispiel, wie später noch erläutert werden wird, für jeden der Eingangsdatensätze 1, 2, für die bereits Dateien 7, 8 beziehungsweise Teildaten empfangen wurden, ein separater Ordner 30, 31 im Dateisystem 16 des Zielsystems 15 angelegt wird, ist es hierfür ausreichend, zu prüfen, ob für die Kennung 42 bereits ein Ordner 30, 31 vorhanden ist. Ist dies nicht der Fall, wird eine Initialisierungsfunktion 29 ausgeführt, die im Beispiel die Schritte S3, S4 und S5 implementiert.

Hierbei wird in Schritt S3 zunächst der Ordner 30, 31 für den Eingangsdatensatz 1, 2, zu dem erstmals eine Datei 7, 8 beziehungsweise Teildaten empfangen wurden, angelegt.

In Schritt S4 wird ein Initialisierungshinweis 32 an den Benutzer 24 ausgegeben, beispielsweise an eine ihm zugeordnete Kommunikationseinrichtung 25 oder E-Mail-Adresse gesendet, um den Benutzer 24 zu informieren, dass Dateien 7, 8 beziehungsweise Teildaten bezüglich der Analyse einer der Proben 3-6 empfangen wurden, wodurch implizit auch der Beginn der Analyse der jeweiligen Probe 3-6 bestätigt wird. Das Ziel des Initialisierungshinweises 32 kann beispielsweise als Teil der Dateien 7, 8, beispielsweise als Header, übertragen werden, sodass es beispielsweise auch möglich ist, den Initialisierungshinweis 32, je nachdem, welche Probe 3-6 analysiert wird beziehungsweise je nach Quellsystemen 9-12, an unterschiedliche Benutzer 24 beziehungsweise Einrichtungen zu senden.

In Schritt S5 wird ein aktueller Zeitpunkt als Referenzzeitpunkt 33 festgelegt, der später, in Schritt S11, ausgewertet werden kann, um zu erkennen, wenn die Übertragung des jeweiligen Eingangsdatensatzes 1, 2 anscheinend ungewöhnlich lange dauert, was auf einen Fehler hinweisen kann. Zudem wird nach Schritt S5 die Überwachung der Übertragung des jeweiligen Eingangsdatensatzes 1, 2 durch die Schritt S7 und die hierauf folgenden Schritte ausgelöst. Dies kann beispielsweise durch das Starten eines parallelen Threads erfolgen.

Unabhängig davon, ob es sich bei der in Schritt S1 empfangenen Datei 7, 8 um die erste Datei 7, 8 eines Eingangsdatensatzes 1, 2 handelt, wird die jeweilige Datei 7, 8 in Schritt S6 in dem dem jeweiligen Eingangsdatensatz 1, 2 zugeordneten Ordner abgelegt.

Die Überwachung, ob die Übertragung des jeweiligen Eingangsdatensatz 1, 2 bereits abgeschlossen ist, beginnt damit, dass in Schritt S7 zunächst für eine vorgegebene Wartezeit 22 gewartet wird, wonach in Schritt S8 zunächst eine bisherige Dateianzahl 18 der in dem Dateisystem 16 beziehungsweise in dem jeweiligen Ordner 30, 31 gespeicherten Dateien 7, 8 des jeweiligen Eingangsdatensatzes 1, 2 ermittelt wird.

Anschließend wird in Schritt S9 erneut für die vorgegebene Wartezeit 22 gewartet und in Schritt S10 anschließend eine aktuelle Dateianzahl 20 der in dem Dateisystem 16 beziehungsweise in dem jeweiligen Ordner 30, 31 gespeicherten Dateien 7, 8 des jeweiligen Eingangsdatensatzes 1, 2 ermittelt.

In Schritt S 11 wird geprüft, ob eine Zeitüberschreitung vorliegt, also ob der jeweilige Eingangsdatensatz 1, 2 trotz des Verstreichens von langer Zeit seit Beginn der Übertragung beziehungsweise der Erfassung des Referenzzeitpunkt 33 anscheinend noch nicht vollständig empfangen wurde. Wird in Schritt S11 festgestellt, dass ein vorgegebener Zeitabstand 35 von beispielsweise mehreren Stunden oder einem Tag zu dem Referenzzeitpunkt 33 überschritten wird, so wird anschließend in Schritt S12 ein Fehlerhinweis 34 ausgegeben, der beispielsweise an den Benutzer 24 oder einen anderen Benutzer, beispielsweise einen Techniker, gerichtet sein kann, um auf die anscheinend fehlgeschlagene Übertragung hinzuweisen.

Im Beispiel sind die Schritte S11 und S12 aus Übersichtlichkeitsgründen in die Schleife 19 integriert. Wie bereits im allgemeinen Teil erläutert wurde, kann es jedoch vorteilhaft sein, diese Schritte parallel zur Schleife 19, beispielsweise in einem separaten Thread, durchzuführen, um beispielsweise auch eine Fehlfunktion des die Schleife 19 implementierenden Programmteils beziehungsweise Threads erkennen zu können.

Erfolgt kein Abbruch der Schleife 19 aufgrund der Erkennung eines Fehlers in Schritt S 11, so wird in Schritt S13 eine Wiederholungsbedingung 21 geprüft, die nur dann erfüllt wird, wenn die aktuelle Dateianzahl 20 die bisherige Dateianzahl 18 überschreitet, also wenn seit der Ermittlung der bisherigen Dateianzahl 18 in Schritt S8 wenigstens eine weitere Datei 7, 8 des jeweiligen Eingangsdatensatzes 1, 2 empfangen wurde. Ist dies der Fall, so war die Übertragung des jeweiligen Eingangsdatensatzes 1, 2 zumindest im aktuellen Durchlauf der Schleife 19 noch nicht abgeschlossen, sodass die Schleife 19 nach der Wartezeit 22 erneut wiederholt wird. Beim nachfolgenden Durchlauf der Schleife 19 wird jedoch die zuletzt in Schritt S10 erfasste aktuelle Dateianzahl 20 nun als bisherige Dateianzahl 18 verwendet, sodass bei einem jeweiligen Durchlauf des Schritts S13 jeweils die in den vorangehenden beiden Wiederholungen des Schritts S10 erfassten Dateianzahlen 20 miteinander verglichen werden.

Wird in Schritt S 13 hingegen ermittelt, dass die aktuelle Dateianzahl 20 identisch zur bisherigen Dateianzahl 18 ist und sich somit die Dateianzahl 18, 20 seit der letzten Wiederholung der Schleife 19 nicht erhöht hat, so kann angenommen werden, dass die Übertragung des jeweiligen Eingangsdatensatzes 1,2 abgeschlossen ist, da für eine hinreichend lange Wartezeit 22 keine weiteren Dateien 7, 8 empfangen beziehungsweise gespeichert wurden.

In dem in Fig. 1 gezeigten Beispiel wird dann in Schritt S14 ein den Abschluss der Übertragung des jeweiligen Eingangsdatensatzes 1, 2 betreffender Abschlusshinweis 23 an eine Freigabeeinrichtung 26 gegeben, der im Beispiel die zuletzt erfasste aktuelle Dateianzahl 20 sowie ein Datenvolumen 36 des jeweiligen Eingangsdatensatzes 1, 2, also die Summe der Datenvolumen der einzelnen Dateien 6, 7 dieses Eingangsdatensatz 1,2 beziehungsweise im zugeordneten Ordner 30, 31 umfasst.

In Schritt S15 wird dann durch die Freigabeeinrichtung 26 eine Freigabebedingung 37 auswertet, die nur dann erfüllt wird, wenn die zuletzt erfasste aktuelle Dateianzahl 20 und das Datenvolumen 36 innerhalb eines jeweiligen vorgegebenen Erwartungsintervalls 38 liegen. Hierdurch kann beispielsweise erkannt werden, wenn die Dateianzahl 18, 20 aufgrund eines Übertragungsabbruchs beziehungsweise Übertragungsfehlers konstant bleibt und somit dieser Umstand nicht auf eine vollständige Übertragung des Eingangsdatensatzes 1, 2 hinweist.

Wenn die Freigabebedingung 37 erfüllt ist, gibt die Freigabeeinrichtung 26 die Datenverarbeitung 27 des jeweiligen Eingangsdatensatz 1, 2 frei, beispielsweise indem eine entsprechende Nachricht an das Zielsystem 15 übermittelt wird. Nach der Datenverarbeitung 27 in Schritt S16 kann das Verarbeitungsergebnis beispielsweise an ein Clientensystem 43 übertragen werden.

Bei Nichterfüllung der Freigabebedingung 37 kann hingegen in Schritt S 17 eine Fehlerbehandlung erfolgen, beispielsweise indem eine Fehlerinformationen an den Benutzer 24 übermittelt wird.

Statt einer automatisierten Freigabe durch die Freigabeeinrichtung 26 kann beispielsweise eine manuelle Freigabe durch den Benutzer 24 erfolgen. Zu diesem Zweck könnte als Abschlusshinweis 23 beispielsweise eine E-Mail an den Benutzer gesendet werden, wonach dieser die Datenverarbeitung 27 manuell freigeben kann.

In einer alternativen Ausgestaltung können die Schritte S14 bis S16 durch die Schritte S14' und S15' ersetzt werden. In Schritt S14', kann dann unmittelbar durch das Zielsystem 15 eine Zusatzbedingung 28 geprüft werden, deren Erfüllung davon abhängt, ob die zuletzt erfasste aktuelle Dateianzahl 20 und das Datenvolumen 36 des jeweiligen Eingangsdatensatzes 1, 2 innerhalb eines jeweiligen vorgegebenen Erwartungsintervalls 38 liegen. Ist dies der Fall, kann unmittelbar in Schritt S15 die Datenverarbeitung 27 für den jeweiligen Eingangsdatensatz 1, 2 ausgelöst werden. Anderenfalls kann wiederum eine Fehlerbehandlung in Schritt S17 erfolgen. Die Prüfung der Zusatzbedingung 28 kann somit im Wesentlichen der obig erläuterten Prüfung der Freigabebedingung 37 entsprechen, wobei die Prüfung jedoch nicht notwendig durch eine externe Einrichtung erfolgen muss, sondern durch das Zielsystem 15 selbst erfolgen kann.

Das erläuterte Verfahren beziehungsweise die durch das Zielsystem 15 durchgeführten Schritte dieses Verfahrens sind im Beispiel dadurch implementiert, dass eine programmierbare Datenverarbeitungseinrichtung 44 genutzt wird, in deren Dateisystem 16 zusätzlich ein Computerprogramm 39 abgelegt ist, dass die Verfahrensschritte implementiert, wenn es durch den Prozessor 40 der Datenverarbeitungseinrichtung 44 ausgeführt wird. Die Kommunikation mit der Freigabeeinrichtung 26 und dem Clientensystem 43 erfolgt im Beispiel über ein Netzwerk 41, vorzugsweise ein virtuelles privates Netzwerk. Die Kommunikation mit dem Benutzer 24 kann drahtlos beziehungsweise beispielsweise per E-Mail oder über einen Nachrichtendienst erfolgen.

Unabhängig vom grammatikalischen Geschlecht eines bestimmten Begriffes sind Personen mit männlicher, weiblicher oder anderer Geschlechteridentität mit umfasst.

### Bezugszeichenliste

- 1, 2: Eingangsdatensatz
- 3-6: Probe
- 7, 8: Datei
- 9-12: Quellsystem
- 13, 14: Labor
- 15: Zielsystem
- 16: Dateisystem
- 17: Pfeil
- 18: Dateianzahl
- 19: Schleife
- 20: Dateianzahl
- 21: Wiederholungsbedingung
- 22: Wartezeit
- 23: Abschlusshinweis
- 24: Benutzer
- 25: Kommunikationseinrichtung
- 26: Freigabeeinrichtung
- 27: Datenverarbeitung
- 28: Zusatzbedingung
- 29: Initialisierungsfunktion
- 30, 31: Ordner
- 32: Initialisierungshinweis
- 33: Referenzzeitpunkt
- 34: Fehlerhinweis
- 35: Zeitabstand
- 36: Datenvolumen
- 37: Freigabebedingung
- 38: Erwartungsintervall
- 39: Computerprogramm
- 40: Prozessor
- 41: Netzwerk
- 42: Kennung
- 43: Clientensystem
- 44: Datenverarbeitungseinrichtung
- S1-S17: Schritt
- S14', S15': Schritt

## Patentansprüche

1. Computerimplementiertes Verfahren zur Steuerung einer Datenverarbeitung (27) wenigstens eines Eingangsdatensatzes (1, 2), der insbesondere auf einer Analyse einer biologischen und/oder medizinischen Probe (3-6) basiert, wobei der jeweilige Eingangsdatensatz (1, 2) mehrere separate Dateien (7, 8) umfasst, wobei eine Übertragung des jeweiligen Eingangsdatensatzes (1, 2) von einem oder einem jeweiligen Quellsystem (9-12) zu einem Zielsystem (15) erfolgt und die Dateien (7, 8) des jeweiligen übertragenen Eingangsdatensatzes (1, 2) durch das Zielsystem (15) in einem Dateisystem (16) des Zielsystems (15) gespeichert werden,
**dadurch gekennzeichnet, dass** durch das Zielsystem (15) nach dem Beginn der Übertragung eine bisherige Dateianzahl (18) der in dem Dateisystem (16) gespeicherten Dateien (7, 8) des jeweiligen Eingangsdatensatzes (1, 2) ermittelt wird, wonach in einer Schleife (19) die folgenden Schritte für den jeweiligen Eingangsdatensatz (1, 2) wiederholt werden:
- Ermitteln einer aktuellen Dateianzahl (20) der in dem Dateisystem (16) gespeicherten Dateien (7, 8) des jeweiligen Eingangsdatensatzes (1, 2),
- Prüfen einer Wiederholungsbedingung (21), die erfüllt wird oder ausschließlich erfüllbar ist, wenn die aktuelle Dateianzahl (20) die bisherige Dateianzahl (18) überschreitet, und
- Wiederholen der Schleife (19) nach einer vorgegebenen Wartezeit (22), wenn die Wiederholungsbedingung (21) erfüllt ist, wobei in dem nachfolgenden Schleifendurchlauf als bisherige Dateianzahl (18) die in dem aktuellen Schleifendurchlauf oder die in einem früheren Schleifendurchlauf erfasste aktuelle Dateianzahl (20) verwendet wird,
wobei bei Nichterfüllung der Wiederholungsbedingung (21),
- entweder ein den Abschluss der Übertragung betreffender Abschlusshinweis (23) an einen Benutzer (24) oder eine Freigabeeinrichtung (26) gegeben wird, wobei nach einer Freigabe durch den Benutzer (24) oder die Freigabeeinrichtung (26) die Datenverarbeitung (27) des jeweiligen Eingangsdatensatzes (1, 2) begonnen wird, oder
- entweder stets oder bei Erfüllung einer Zusatzbedingung (28) die Datenverarbeitung (27) des jeweiligen Eingangsdatensatzes (1, 2) begonnen wird.

2. Computerimplementiertes Verfahren nach Anspruch 1, wobei eine Steuerung der Datenverarbeitung (27) mehrerer Eingangsdatensätze (1, 2) erfolgt, **dadurch gekennzeichnet, dass** seitens des Zielsystems (15) jeweils eine Initialisierungsfunktion (29) ausgeführt wird, wenn erkannt wird, dass erstmalig eine Datei (7, 8) oder Teildaten einer Datei (7, 8) eines der Eingangsdatensätze (1, 2) empfangen werden.

3. Computerimplementiertes Verfahren nach Anspruch 2, **dadurch gekennzeichnet, dass** im Rahmen der Initialisierungsfunktion (29) ein dem jeweiligen Eingangsdatensatz (1, 2) zugeordneter Ordner (30, 31) in dem Dateisystem (16) angelegt wird, wobei die Dateien (7, 8) des jeweiligen Eingangsdatensatzes (1, 2) in diesem Ordner (30, 31) abgelegt werden.

4. Computerimplementiertes Verfahren nach Anspruch 2 oder 3, **dadurch gekennzeichnet, dass** im Rahmen der Initialisierungsfunktion (29) ein Initialisierungshinweis (32) an den Benutzer (24) und/oder einen weiteren Benutzer und/oder die Freigabeeinrichtung (26) und/oder eine andere Einrichtung gegeben wird.

5. Computerimplementiertes Verfahren nach einem der Ansprüche 2 bis 4, **dadurch gekennzeichnet, dass** im Rahmen der Initialisierungsfunktion (29) ein aktueller Zeitpunkt als Referenzzeitpunkt (33) festgelegt wird, wobei ein Fehlerhinweis (34) an den Benutzer (24) und/oder den oder einen weiteren Benutzer und/oder die Freigabeeinrichtung (26) und/oder die oder eine andere Einrichtung gegeben wird, wenn ein Zeitabstand (35) zu dem Referenzzeitpunkt (33) überschritten wird, bevor der Abschlusshinweis (23) gegeben und/oder die Datenverarbeitung (27) des jeweiligen Eingangsdatensatzes (1, 2) begonnen wird.

6. Computerimplementiertes Verfahren nach einem der vorangehenden Ansprüche, **dadurch gekennzeichnet, dass** der Abschlusshinweis (23) die zuletzt erfasste aktuelle Dateianzahl (20) und/oder ein Datenvolumen (36) des jeweiligen Eingangsdatensatzes (1, 2) umfasst.

7. Computerimplementiertes Verfahren nach Anspruch 6, **dadurch gekennzeichnet, dass** die Freigabeeinrichtung (26) eine Freigabebedingung (37) auswertet, deren Erfüllung davon abhängt, ob die zuletzt erfasste aktuelle Dateianzahl (20) und/oder das Datenvolumen (36) innerhalb eines jeweiligen vorgegebenen Erwartungsintervalls (38) liegen.

8. Computerimplementiertes Verfahren nach einem der Ansprüche 1 bis 5, **dadurch gekennzeichnet, dass** die Erfüllung der Zusatzbedingung (28) davon abhängt, ob die zuletzt erfasste aktuelle Dateianzahl (20) und/oder ein Datenvolumen (36) des jeweiligen Eingangsdatensatzes (1, 2) innerhalb eines jeweiligen vorgegebenen Erwartungsintervalls (38) liegen.

9. Computerimplementiertes Verfahren nach einem der vorangehenden Ansprüche, **dadurch gekennzeichnet, dass** der jeweilige Eingangsdatensatz (1, 2) durch eine Analyse einer biologischen und/oder medizinischen Probe (3-6) durch das Quellsystem (9-12) ermittelt wird, wobei die Übertragung des jeweiligen Eingangsdatensatzes (1, 2) beginnt, bevor die Analyse der biologischen und/oder medizinischen Probe (3-6) abgeschlossen ist.

10. Computerimplementiertes Verfahren nach einem der vorangehenden Ansprüche, **dadurch gekennzeichnet, dass** die Eingangsdaten (1, 2) durch Sequenzierung wenigstens eines Teils wenigstens eines Genoms in einer biologischen und/oder medizinischen Probe (3-6) bereitgestellt werden.

11. Datenverarbeitungseinrichtung, **dadurch gekennzeichnet, dass** sie zur Teilnahme an dem computerimplementierten Verfahren nach einem der vorangehenden Ansprüche als das Zielsystem (15) eingerichtet ist.

12. Computerprogramm mit Anweisungen, die dazu eingerichtet sind, bei einer Ausführung auf einer Datenverarbeitungseinrichtung (44) die durch das Zielsystem (15) implementierten Schritte des computerimplementierten Verfahrens nach einem der Ansprüche 1 bis 10 durchzuführen.

13. Datenträger umfassend ein Computerprogramm (39) nach Anspruch 12.
